# EUROPÄISCHE PATENTSCHRIFT

(11) **EP 2 435 106 B1**
(45) Veröffentlichungstag und Bekanntmachung des Hinweises auf die Patenterteilung: **26.11.2014**
(21) Anmeldenummer: 10715829.7
(22) Anmeldetag: 26.04.2010
(51) Int. Cl.: A61M 1/36

(54) **VORRICHTUNG ZUR FESTLEGUNG DES VENÖSEN ZUFLUSSES ZU EINEM BLUTRESERVOIR EINES EXTRAKORPORALEN BLUTKREISLAUFS**
DEVICE FOR ESTABLISHING THE VENOUS INFLOW TO A BLOOD RESERVOIR OF AN EXTRACORPOREAL BLOOD CIRCULATION SYSTEM
DISPOSITIF POUR LA DÉTERMINATION DE L'AFFLUX DE SANG VEINEUX VERS UN RÉSERVOIR DE SANG POUR D'UNE CIRCULATION EXTRACORPORELLE

(30) Priorität: 29.05.2009 DE 102009026592
(43) Veröffentlichungstag der Anmeldung: 04.04.2012
(60) Teilanmeldung: 14188440.3
(73) Patentinhaber: Sorin Group Deutschland GmbH, 80939 München (DE)
(72) Erfinder: KNOTT, Erwin, 85586 Poing (DE); SCHREYER, Johann, 80999 München (DE)
(74) Vertreter: Hoffmann Eitle
(86) Internationale Anmeldenummer: PCT/EP2010/055522
(87) Internationale Veröffentlichungsnummer: WO 2010/136283

(56) Entgegenhaltungen:
- WO-A2-01/76656
- WO-A2-96/24397
- US-A1- 2006 015 056

## Beschreibung

Die Erfindung betrifft eine Vorrichtung zur Festlegung des venösen Zuflusses zu einem Blutreservoir in einem extrakorporalen Blutkreislauf.

Ein extrakorporaler Blutkreislauf umfasst neben anderen Komponenten regelmäßig eine venöse Zuflussleitung von einem Patienten zu einem Blutreservoir, zum Beispiel einem venösen Kardiotomie-Reservoir für Blut und/oder Priminglösung, und eine arterielle Zuflussleitung von dem Reservoir zu dem Patienten. Um das Blut von dem Patienten in das Reservoir zu fördern, befindet sich das Reservoir auf einem Höhenniveau unterhalb des Patienten, so dass die Drainage schon mittels Schwerkraft erfolgen kann. Mit einer Blutpumpe, in der Regel einer Rollerpumpe, wird das Blut aus dem Reservoir durch die arterielle Zuflussleitung gefördert und dem Patienten zugeführt.

Damit der venöse Zufluss zum Reservoir in ausreichendem Maß geschieht, sind bei reiner Schwerkraftdrainage große Schlauchquerschnitte erforderlich, was im Hinblick auf die mit der Drainageleitung in Verbindung stehende Belastung des Patienten problematisch ist. Zudem ist eine reine Schwerkraftdrainage ungünstig im Hinblick auf die Beeinflussbarkeit der venösen Zuflussmenge, da dazu der Höhenunterschied zwischen Reservoir und Patient während der Aufrechterhaltung des extrakorporalen Kreislaufs verändert werden müsste.

Wie zum Beispiel in WO 00/44415 A beschrieben, ist bereits vorgeschlagen worden, das Reservoir mit einem Unterdruck zu beaufschlagen, um den Querschnitt der Drainageleitung reduzieren und den venösen Zufluss beeinflussen zu können. WO 00/44415 A offenbart in diesem Zusammenhang einen Unterdruckregler, der die Handhabung des Unterdrucks durch einen Benutzer vereinfacht, um so die Patientensicherheit während einer Operation zu steigern. Denn durch eine zuverlässige Regelung des Unterdrucks wird zunächst eine einfache Einstellung des Unterdrucks durch den Benutzer und damit eine einfache Einstellung des venösen Zuflusses ermöglicht. Durch eine zuverlässige Regelung des Unterdrucks wird aber auch ein zu hoher und eine Gefährdung des Patienten darstellender Unterdruck vermieden, wobei zu beachten ist, dass durch die Entnahme des Blutes aus dem Reservoir mit Hilfe der Blutpumpe, die das Blut dem Patienten arteriell zuführt, zum Aufbau bzw. zur Erhöhung des Unterdrucks beigetragen wird. In WO 00/44415 A werden zuvor bekannte Lösungen als unzureichend bezeichnet und ein eigenständiges Gerät zur Unterdruckregelung beschrieben, dass zuverlässig den Unterdruck im Reservoir regelt und ggf. auch reduziert, wenn dies erforderlich sein sollte.

Weiterhin beschreibt das Dokument US 2006/015056 A1 eine Vorrichtung zur Festlegung des venösen Zuflusses zu einem Blutreservoir eines extrakorporalen Blutkreislaufs, der eine venöse Zuflussleitung von einem Patienten zu dem Reservoir und eine arterielle Zuflussleitung von dem Reservoir zu dem Patienten umfasst, mit einer Drosseleinrichtung zum graduellen Verschließen der venösen Zuflussleitung, um die venöse Zuflussmenge zum Reservoir zu drosseln, einer Unterdruckeinrichtung zur Beaufschlagung des Reservoirs mit einem Unterdruck, um die venöse Zuflussmenge zum Reservoir zu erhöhen und einer Steuereinrichtung, die der Drosseleinrichtung ein erstes Ansteuersignal zur Festlegung des Ausmaßes des Verschließens der venösen Zuflussleitung zuführt, um den Umfang der Drosselung der venösen Zuflussmenge zum Reservoir zu bestimmen, die der Unterdruckeinrichtung ein zweites Ansteuersignal zur Festlegung der Höhe des Unterdrucks in dem Reservoir zuführt, um den Umfang der Erhöhung der venösen Zuflussmenge zum Reservoir zu bestimmen.

Bei der Zuführung des Blutes aus dem Reservoir zum Patienten muss darauf geachtet werden, dass nicht mehr Blut entnommen wird, als im Reservoir vorhanden ist bzw. diesem zugeführt wird. Der Benutzer muss also auf eine ausreichende Mindestmenge im Reservoir achten und zusätzlich Zu- und Abfluss so aufeinander abstimmen, dass ausreichende Mengen im Reservoir aufgebaut aber auch dem Patienten zugeführt werden, da bei einem extrakorporalen Blutkreislauf eine physiologisch ausreichend Versorgung des Patienten immer gewährleistet sein muss. Dabei kann er beispielsweise die Fördermenge der Rollerpumpe erhöhen oder verringern oder er kann den Leitungsquerschnitt der Zuführleitung durch Klemmen verringern bzw. wieder vergrößern. Daneben muss der Benutzer auf die drainierte Blutmenge achten und den Unterdruck im Reservoir - auch bei dem zuvor geschilderten Unterdruckregler- geeignet einstellen. Dieser Anforderung muss der Benutzer in einem Umfeld gerecht werden, das ihn ferner zwingt, bei all seinen Handlungen den durch die extrakorporale Zirkulation unterstützen operativen Eingriff im Blick zu behalten. Insgesamt spielt sich somit die Einstellung der venösen Zuflussmenge zum Reservoir eines extrakorporalen Kreislauf in einem belastenden Arbeitsumfeld ab, so dass dies bisherigen technischen Lösungen bei der Unterstützung des Benutzer bei der Einstellung der venösen Zuflussmenge zum Reservoir als nicht optimal angesehen werden müssen.

Das von der Erfindung zu lösende technische Problem besteht vor diesem Hintergrund darin, eine Vorrichtung zur Festlegung des venösen Zuflusses zu einem Blutreservoir eines extrakorporalen Blutkreislaufs anzugeben, die die Handhabung des extrakorporalen Blutkreislaufs für den Benutzer weiter vereinfacht.

Gelöst wird diese Aufgabe durch eine Vorrichtung, wie sie in Patentanspruch 1 beschrieben ist. Vorteilhafte Ausgestaltungen ergeben sich aus den Unteransprüchen.

Eine erfindungsgemäße Vorrichtung zur Festlegung des venösen Zuflusses zu einem Blutreservoir eines extrakorporalen Blutkreislaufs, der eine venöse Zuflussleitung von einem Patienten zu dem Reservoir und eine arterielle Zuflussleitung von dem Reservoir zu dem Patienten umfasst, weist neben einer Drosseleinrichtung zum graduellen Verschließen, insbesondere Klemmen, der venösen Zuflussleitung, um die venöse Zuflussmenge zum Reservoir zu drosseln, und einer Unterdruckeinrichtung zur Beaufschlagung des Reservoirs mit einem Unterdruck, um die venöse Zuflussmenge zum Reservoir zu erhöhen, ferner eine Steuereinrichtung auf, die der Drosseleinrichtung ein erstes Ansteuersignal zur Festlegung des Ausmaßes der Verschließens der venösen Zuflussleitung zuführt, um den Umfang der Drosselung der venösen Zuflussmenge zum Reservoir zu bestimmen, die der Unterdruckeinrichtung ein zweites Ansteuersignal zur Festlegung der Höhe des Unterdrucks in dem Reservoir zuführt, um den Umfang der Erhöhung der venösen Zuflussmenge zum Reservoir zu bestimmen, und die ein einziges Bedienelement zur Festlegung der Menge des venösen Zuflusses zum Reservoir durch einen Bediener aufweist.

Durch Bereitstellung ein einziges Bedienelement zur Festlegung der Menge des venösen Zuflusses zum Reservoir durch einen Bediener wird dem Benutzer auf einfache Weise die Möglichkeit gegeben den venösen Zufluss zum Reservoir sowohl oberhalb als auch unterhalb eines Basiswertes mit einem einzigen Einstellvorgang zu bestimmen. Der Basiswert wird dadurch festgelegt, dass für venöse Zuflussmengen unterhalb dieses Wertes eine Drosselung der Förderung und für venöse Zuflussmengen oberhalb dieses Wertes eine aktive Unterstützung der Förderung der um die ansonsten durch die Schwerkraft geförderten und definierten Zuflussmenge handelt. Durch die erfindungsgemäße Gestaltung der Steuereinrichtung wird erreicht, dass die erfindungsgemäße Vorrichtung den venösen Zufluss entsprechend einstellt und regelt. Dabei ist zu beachten, dass sich das zuvor und auch im Folgenden beschriebene einzige Bedienelement allein auf die Funktion der Festlegung der Menge des venösen Zuflusses zum Reservoir durch einen Bediener bezieht. Daneben kann die Steuereinrichtung weitere Bedienelemente aufweisen, wie zum Beispiel einen Ein/Aus-Schalter, einen Helligkeitsregler für ggf. vorhandene Anzeigen, einen Wahlschalter zur Aktivierung/Deaktivierung eines Alarms usw. Erfindungsgemäß ist aber zur Festlegung der Menge des venösen Zuflusses zum Reservoir durch einen Bediener nur ein einziges Bedienelement vorgesehen, das die Festlegung des venösen Zuflusses sowohl oberhalb als auch unterhalb eines Basiswertes erlaubt.

In einer vorteilhaften Ausgestaltung umfasst die Steuereinrichtung eine Anzeigeeinrichtung für die visuelle Anzeige eines der Menge des venösen Zuflusses entsprechenden Anzeigewerts. Mittels dieser Anzeige kann dem Benutzer eine Wertangabe bezüglich des venösen Zuflusses, des Unterdrucks im Reservoir und/oder der im Reservoir bevorrateten Blutmenge angezeigt werden.

In einer vorteilhaften Ausgestaltung ist die Unterdruckeinrichtung über eine Leitung an eine Unterdruckquelle angeschlossen oder umfasst alternativ bzw. zusätzlich eine integrierte Unterdruckquelle, insbesondere eine Pumpe.

In einer besonders vorteilhaften Ausgestaltung ist ein Unterdrucksensor zur Erfassung des in dem Reservoir vorhanden Unterdrucks vorgesehen, der mit der Steuereinrichtung zur Bereitstellung eines entsprechende Messsignals verbunden ist.

In einer besonders vorteilhaften Ausgestaltung ist ein Pegelsensor zur Erfassung des Pegels der in dem Reservoir vorhandenen Blutmenge vorgesehen, der mit der Steuereinrichtung zur Bereitstellung eines entsprechenden Messsignals verbunden ist.

In einer besonders vorteilhaften Ausgestaltung ist ein arterieller Zuflussmengensensor zur Erfassung der in der arteriellen Zuflussleitung geförderte Zuflussmenge vorgesehen, der mit der Steuereinrichtung zur Bereitstellung eines entsprechenden Messsignals verbunden ist.

In einer besonders vorteilhaften Ausgestaltung erzeugt die Steuereinrichtung ein die Fördermenge einer Blutpumpe festlegendes drittes Ansteuersignal, so dass die arterielle Zuflussmenge zumindest beeinflusst werden kann.

In einer vorteilhaften Ausgestaltung ist das einzige Bedienelement für die Vorgabe einer im Reservoir zu bevorratenden Blutmenge ausgelegt, so dass auf diese Weise eine Festlegung des venösen Zufluss zum Reservoir erfolgen kann.

In einer vorteilhaften Ausgestaltung umfasst die Unterdruckeinrichtung eine Sicherheitseinrichtung, die auf Ansteuerung durch die Steuereinrichtung hin oder bei Versagen der Steuerung die Beaufschlagung des Reservoirs mit Unterdruck unterbricht und Umgebungsdruck im Reservoir herstellt.

Im Folgenden wird die Erfindung unter Bezugnahme auf die Zeichnungen genauer beschrieben, in denen zeigt:
- Figur 1: eine Skizze des prinzipiellen Aufbau eines extrakorporalen Blutkreislaufs mit einer Vorrichtung gemäß einem ersten Ausführungsbeispiel der Erfindung;
- Figur 2: eine Skizze des prinzipiellen Aufbau eines extrakorporalen Blutkreislaufs mit einer Vorrichtung gemäß einem zweiten Ausführungsbeispiel der Erfindung;
- Figur 3: eine Skizze des prinzipiellen Aufbau eines extrakorporalen Blutkreislaufs mit einer Vorrichtung gemäß einem dritten Ausführungsbeispiel der Erfindung; und
- Figur 4: eine Skizze des prinzipiellen Aufbau eines extrakorporalen Blutkreislaufs mit einer Vorrichtung gemäß einem vierten Ausführungsbeispiel der Erfindung.

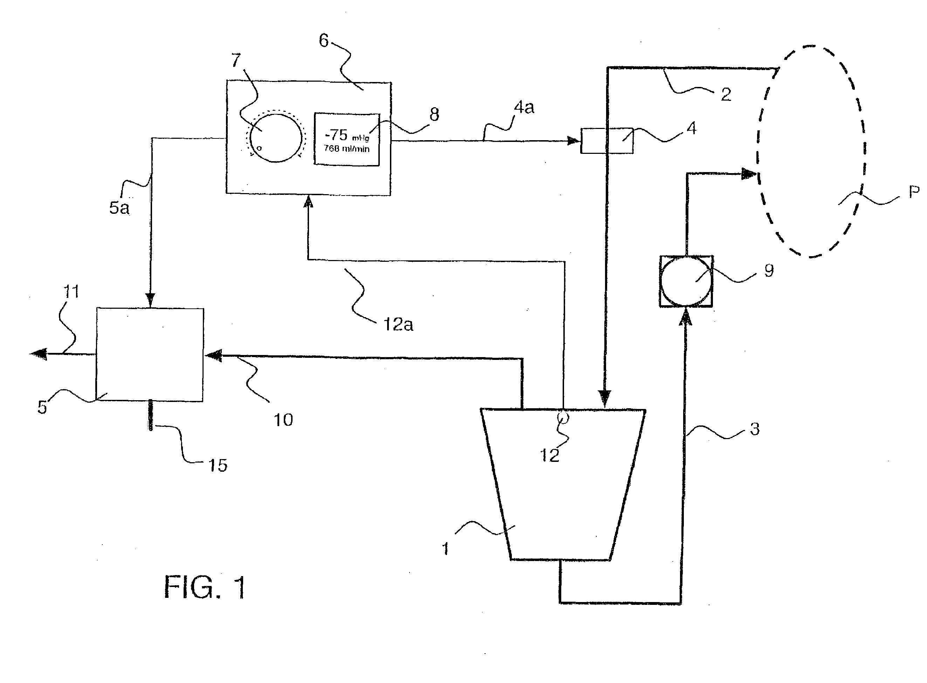

Wie in Figur 1 anhand eines erstes Ausführungsbeispiels einer Vorrichtung gemäß der Erfindung gezeigt, umfasst ein extrakorporaler Blutkreislauf prinzipiell neben einem Reservoir 1 eine venöse Zuflussleitung 2 von einem Patienten P zu dem Reservoir 1 und eine arterielle Zuflussleitung 3 von dem Reservoir 1 zu dem Patienten P. Für die Zuführung des Blutes aus dem Reservoir 1 zum Patienten P ist eine Pumpe 9, in der Regel eine Rollerpumpe oder eine Zentrifugalpumpe, an bzw. in der arteriellen Zuflussleitung 3 vorgesehen. Wie in Figur 1 erkennbar, ist das Reservoir 1 auf einem Höhenniveau unterhalb des Patienten angeordnet, so dass bereits allein aufgrund der Schwerkraft ein venöser Zufluss zum Reservoir möglich ist, dessen Ausmaß aber auch vom Querschnitt des im venösen Zufluss eingesetzten Schlauches abhängt.

Um den venösen Zufluss zu dem Reservoir 1 zu drosseln, umfasst das erste hier beschriebene Ausführungsbeispiel der Erfindung eine Drosseleinrichtung 4, mit der die venöse Zuflussleitung 2, zum Beispiel durch Klemmen oder Quetschen, graduell verschlossen werden kann. Dazu weist die Drosseleinrichtung 4 eine in Figur 1 nicht im Einzelnen gezeigte elektromagnetisch, pneumatisch oder hydraulisch betätigte Klemme auf. Die Drosseleinrichtung 4 ist an der venösen Zuflussleitung 2 angeordnet und wirkt vorteilhafterweise von außen auf den Schlauch ein, ohne mit dem Blut in Kontakt zu geraten.

Um den venösen Zufluss zu dem Reservoir 1 zu erhöhen, umfasst das erste Ausführungsbeispiel einer erfindungsgemäßen Vorrichtung eine Unterdruckeinrichtung 5 zur Beaufschlagung des Reservoirs 1 mit einem Unterdruck. Dazu ist die Unterdruckeinrichtung 5 über eine Unterdruckleitung 10 mit dem entsprechend druckdicht gestalteten Reservoir 1 verbunden. Sofern die Unterdruckeinrichtung 5 nicht selbst für die Erzeugung des Unterdrucks, beispielsweise durch Integration einer geeigneten Pumpe, ausgelegt ist, ist die Unterdruckeinrichtung 5 wie bei dem in Figur 1 gezeigten ersten Ausführungsbeispiel mit einer Unterdruckquelle über eine Unterdruckquellenleitung 11 verbunden. Geeignete Unterdruckquellen sind in Krankenhäusern, in denen die erfindungsgemäße Vorrichtung vorrangig einsetzbar ist, in Form von ortsfesten Installationen in der Regel vorhanden.

Gemäß der Erfindung erfolgt die Festlegung des venösen Zuflusses durch den Benutzer auf einfache und komfortable aber gleichzeitig auch sichere Weise über eine Steuereinrichtung 6, die in Figur 1 gezeigt ist. Die Steuereinrichtung 6 führt der Drosseleinrichtung 4 über eine erste Ansteuerleitung 4a ein erstes Ansteuersignal zur Festlegung des Ausmaßes der Verschließens der venösen Zuflussleitung 2 zu, um die Drosselung der venösen Zuflussmenge zum Reservoir vorzunehmen. Ferner führt die Steuereinrichtung 6 der Unterdruckeinrichtung 5 über eine zweite Ansteuerleitung 5a ein zweites Ansteuersignal zur Festlegung der Höhe des Unterdrucks in dem Reservoir 1 zu, um die Erhöhung der venösen Zuflussmenge zum Reservoir vorzunehmen.

Erfindungsgemäß umfasst die Steuereinrichtung 6 ein einziges Bedienelement 7 zur Festlegung der Menge des venösen Zuflusses zum Reservoir durch einen Bediener der Vorrichtung. Allein durch Betätigung dieses einen Bedienelements 7 kann der Benutzer die venöse Zuflussmenge zum Reservoir 1 festlegen und dabei sowohl eine Erhöhung über die im Wesentlichen durch die Schwerkraftdrainage und den auf der venösen Seite eingesetzten Schlauchquerschnitt bestimmte Menge hinaus als auch eine Reduzierung unter diesen Wert vornehmen. Die erfindungsgemäße Steuereinrichtung 6 setzt die von dem Benutzer mit Hilfe des einzigen Betätigungselements 7 getroffene Festlegung in eine entsprechende Ansteuerung der Drosseleinrichtung 4 bzw. der Unterdruckeinrichtung 5 um, so dass dadurch eine Verringerung der venösen Zuflussmenge durch Verschließen (Klemmen) der venösen Zuflussschlauchleitung 2 oder eine Erhöhung der venösen Zuflussmenge durch Erzeugen eines Unterdrucks im Reservoir 1 zu bewirken.

Wie Figur 1 zeigt umfasst das erste Ausführungsbeispiel einen Drucksensor 12, der an oder in dem Reservoir 1 so angeordnet ist, dass er den Unterdruck im Reservoir 1 erfasst und ein entsprechendes erstes Messsignal bereitstellt. Das Messsignal des Unterdrucksensors 12 wird über eine erste Messsignalleitung 12a der Steuereinrichtung 6 zugeführt, die die Unterdruckregelung unter Einbeziehung dieses Messsignals vornehmen kann.

Die Steuereinrichtung 6 gemäß dem ersten Ausführungsbeispiel umfasst ferner eine Anzeige 8, die dem Benutzer die eingestellte venöse Zuflussmenge und/oder den eingestellten Unterdruck anzeigt.

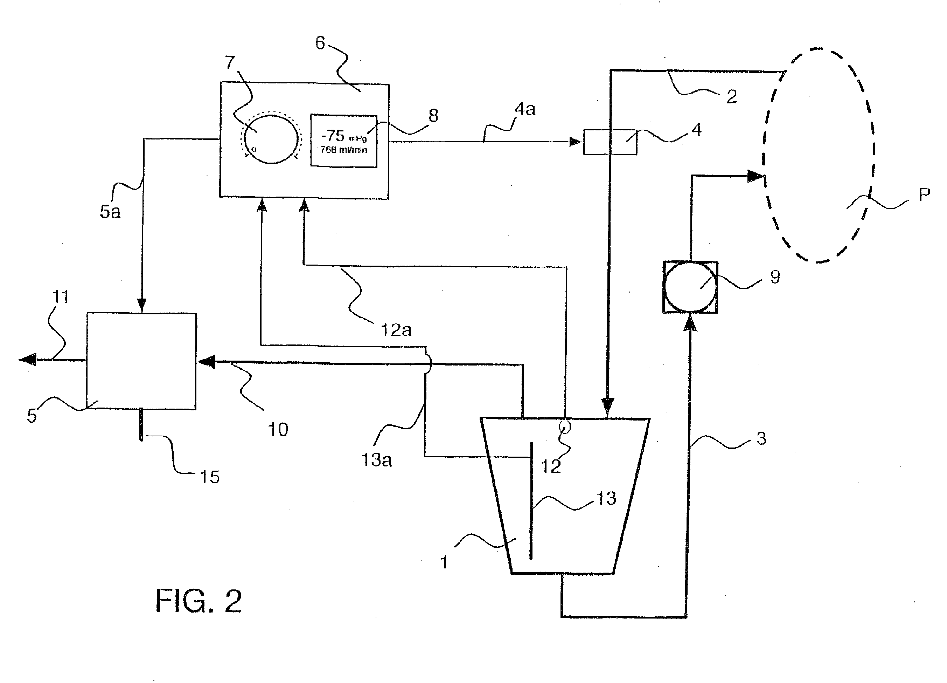

Figur 2 zeigt ein zweites Ausführungsbeispiel einer erfindungsgemäßen Vorrichtung, das alle Aspekte des ersten Ausführungsbeispiels umfasst, so dass hinsichtlich dieser Punkte auf die Beschreibung des ersten Ausführungsbeispiels im Zusammenhang mit Figur 1 verwiesen werden kann. Das zweite Ausführungsbeispiel umfasst zusätzlich einen Pegelsensor 13, der den Pegel des im Reservoir 1 aktuell vorhandenen Blutes und damit die bevorratete Blutmenge erfasst. Der Pegelsensor 13 erzeugt ein zweites Messsignal, das über eine zweite Messsignalleitung 13a der Steuereinrichtung 6 zugeführt wird. Mit Hilfe des zweiten Messsignals ist die Steuereinrichtung 6 in der Lage die aktuell in dem Reservoir 1 vorhandene Blutmenge in die Unterdruckregelung einzubeziehen, um den Unterdruck im Reservoir 1 zu erhöhen und damit den venösen Zufluss zum Reservoir 1 zu steigern, wenn der Pegel des Blutes im Reservoir 1 unter einen vorgegebenen Grenzwert sinkt. Außerdem kann die Steuereinrichtung 6 gemäß dem zweiten Ausführungsbeispiel das Erreichen eines vorgegebenen oberen Füllstands des Reservoirs 1 überwachen, um den venösen Zufluss zu drosseln.

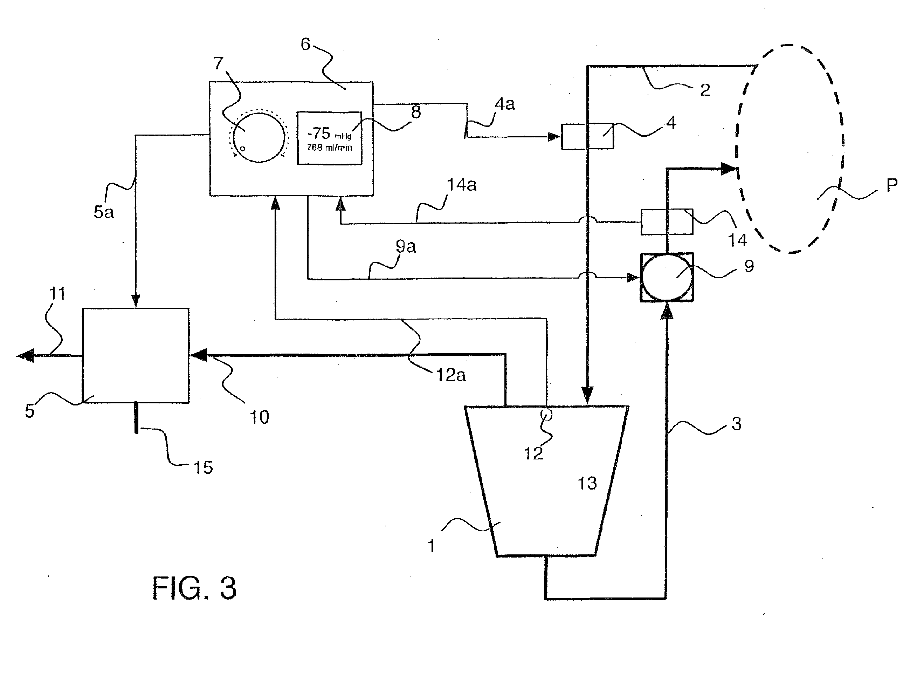

Figur 3 zeigt ein drittes Ausführungsbeispiel einer erfindungsgemäßen Vorrichtung, das alle Aspekte des ersten Ausführungsbeispiels umfasst, so dass hinsichtlich dieser Punkte auf die Beschreibung des ersten Ausführungsbeispiels im Zusammenhang mit Figur 1 verwiesen werden kann. Das dritte Ausführungsbeispiel umfasst zusätzlich einen Zuflussmengensensor 14, der die arterielle Zuflussmenge und damit die Blutmenge erfasst, die dem Patienten P über die arterielle Zuflussleitung 3 zugeführt und damit dem Reservoir 1 entnommen wird. Der Mengensensor 14 ist an der arteriellen Zuflussleitung 3 entweder stromabwärts, wie in Figur 3 gezeigt, oder stromaufwärts von der Pumpe 9 angeordnet. Der Mengensensor 14 gibt ein drittes Messsignal ab, das über eine dritte Messsignalleitung 14a der Steuereinrichtung 6 zugeführt wird. Dadurch ist die Steuereinrichtung 6 in der Lage, bei der Unterdruckregelung die dem Reservoir 1 entnommene Blutmenge zu berücksichtigen und den venösen Zufluss entsprechend zu gestalten. Um auf den arteriellen Zufluss Einfluss nehmen zu können, kann bei dem dritten Ausführungsbeispiel die Steuereinrichtung 6 über eine dritte Ansteuerleitung 9a ein drittes Ansteuersignal an die Pumpe 9 abgeben, wodurch die Fördermenge der Pumpe eingestellt oder zumindest beeinflusst wird.

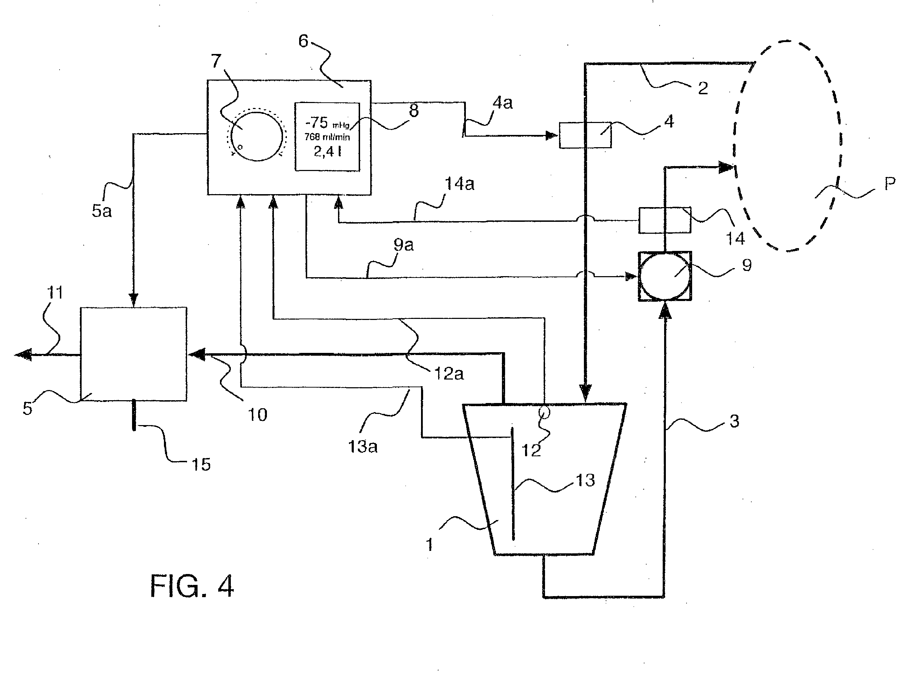

Figur 4 zeigt ein viertes Ausführungsbeispiel einer erfindungsgemäßen Vorrichtung, das alle Aspekte der ersten drei Ausführungsbeispiele umfasst, so dass hinsichtlich dieser Punkte auf die Beschreibung der ersten drei Ausführungsbeispiele im Zusammenhang mit den Figuren 1, 2 und 3 verwiesen werden kann. Durch das Zusammenbringen aller Aspekte der zuvor geschilderten Ausführungsbeispiele wird eine Vorrichtung zur Festlegung des venösen Zuflusses zum Reservoir 1 geschaffen, die eine quasi vollautomatische Steuerung/Regelung der im Reservoir 1 bevorrateten Blutmenge erlaubt. Denn über das graduelle Verschließen der venösen Zuflussleitung und das Regeln des Unterdrucks im Reservoir 1 kann die Steuereinrichtung 6 die dem Reservoir 1 zugeführte Blutmenge festlegen, die sie jeweils aktuell mit Hilfe des Pegelsensors 13 erfasst. Über den Mengensensor 14 kann die Steuereinrichtung 6 gleichzeitig die arterielle Zuflussmenge erfassen und durch Ansteuerung der Pumpe 9 festlegen bzw. beeinflussen. Demzufolge kann das Bedienelement 7 der Steuereinrichtung 6 beim vierten Ausführungsbeispiel auch so ausgelegt sein, dass die im Reservoir 1 bevorratete Blutmenge durch den Benutzer festgelegt wird. In Figur 4 ist diese Ausgestaltung insofern berücksichtigt, als in der Anzeige 8 auch der momentane Füllstand des Reservoirs 1 angezeigt wird. Die Steuereinrichtung 6 bewirkt dann vollautomatisch auf der Basis der Vorgabe der Blutmenge durch den Benutzer und unter Berücksichtigung physiologischer Randbedingungen, die die Versorgung des Patienten sicherstellen, die Einstellung des venösen Zuflusses zum und des arteriellen Abflusses vom Reservoir 1 des extrakorporalen Blutkreislaufs.

Wie in den Figuren 1 bis 4 gezeigt, kann bei allen Ausführungsbeispielen die Unterdruckeinrichtung 5 mit einer Sicherheitseinrichtung 15 ausgestattet sein, die auf entsprechende Ansteuerung von der Steuereinrichtung 6 hin oder bei Versagen der Steuerung die Beaufschlagung des Reservoirs 1 mit Unterdruck unterbricht und Umgebungsdruck im Reservoir 1 herstellt.

## Patentansprüche

1. Vorrichtung zur Festlegung des venösen Zuflusses zu einem Blutreservoir (1) eines extrakorporalen Blutkreislaufs, der eine venöse Zuflussleitung (2) von einem Patienten (P) zu dem Reservoir und eine arterielle Zuflussleitung (3) von dem Reservoir zu dem Patienten umfasst, mit
- einer Drosseleinrichtung (4) zum graduellen Verschließen, insbesondere Klemmen, der venösen Zuflussleitung, um die venöse Zuflussmenge zum Reservoir zu drosseln;
- einer Unterdruckeinrichtung (5) zur Beaufschlagung des Reservoirs mit einem Unterdruck, um die venöse Zuflussmenge zum Reservoir zu erhöhen, und
- einer Steuereinrichtung (6),
∘ die ein einziges Bedienelement (7) zur Festlegung der Menge des venösen Zuflusses zum Reservoir durch einen Bediener oberhalb oder unterhalb eines durch Schwerkraftförderung festgelegten Basiswertes aufweist, und
∘ die aufgrund der Festlegung der Menge des venösen Zuflusses zum Reservoir durch einen Bediener
- der Drosseleinrichtung (4) ein erstes Ansteuersignal zur Festlegung des Ausmaßes der Verschließens der venösen Zuflussleitung zuführt, um den Umfang der Drosselung der venösen Zuflussmenge zum Reservoir zu bestimmen, und
- der Unterdruckeinrichtung (5) ein zweites Ansteuersignal zur Festlegung der Höhe des Unterdrucks in dem Reservoir zuführt, um den Umfang der Erhöhung der venösen Zuflussmenge zum Reservoir zu bestimmen.

2. Vorrichtung nach Anspruch 1, **dadurch gekennzeichnet, dass** die Steuereinrichtung (6) eine Anzeigeeinrichtung (8) für die visuelle Anzeige eines der Menge des venösen Zuflusses entsprechenden Anzeigewerts aufweist.

3. Vorrichtung nach Anspruch 1 oder 2, **dadurch gekennzeichnet, dass** die Unterdruckeinrichtung (5) über eine Leitung (11) an eine Unterdruckquelle angeschlossen ist oder eine Unterdruckquelle, insbesondere eine Pumpe, integriert umfasst.

4. Vorrichtung nach einem der Ansprüche 1, 2 oder 3, **dadurch gekennzeichnet, dass** ein Unterdrucksensor (12) zur Erfassung des in dem Reservoir vorhanden Unterdrucks vorgesehen und mit der Steuereinrichtung (6) zur Bereitstellung eines ersten Messsignals verbunden ist.

5. Vorrichtung nach einem der vorangegangenen Ansprüche, **dadurch gekennzeichnet, dass** ein Pegelsensor (13) zur Erfassung des Pegels der in dem Reservoir (1) vorhandenen Blutmenge vorgesehen und mit der Steuereinrichtung (6) zur Bereitstellung eines zweiten Messsignals verbunden ist.

6. Vorrichtung nach einem der vorangegangenen Ansprüche, **dadurch gekennzeichnet, dass** ein arterieller Zuflussmengensensor (14) zur Erfassung der in der arteriellen Zuflussleitung (3) geförderte Zuflussmenge vorgesehen und mit der Steuereinrichtung (6) zur Bereitstellung eines dritten Messsignals verbunden ist.

7. Vorrichtung nach einem der vorangegangenen Ansprüche, **dadurch gekennzeichnet, dass** eine Blutpumpe (9) vorgesehen ist, der von der Steuereinrichtung (6) ein die Fördermenge der Blutpumpe festlegendes drittes Ansteuersignal zugeführt wird.

8. Vorrichtung nach einem der vorangegangenen Ansprüche, **dadurch gekennzeichnet, dass** das einzige Bedienelement (7) für die Vorgabe einer im Reservoir (1) zu bevorratenden Blutmenge ausgelegt ist.

9. Vorrichtung nach einem der vorangegangenen Ansprüche, **dadurch gekennzeichnet, dass** die Unterdruckeinrichtung (5) eine Sicherheitseinrichtung (15) umfasst, die auf Ansteuerung durch die Steuereinrichtung (6) hin oder bei Versagen der Steuerung die Beaufschlagung des Reservoirs (1) mit Unterdruck unterbricht und Umgebungsdruck im Reservoir (1) herstellt.

## Claims

1. Apparatus for fixing the venous flow to a blood reservoir (1) of an extracorporeal blood circuit which comprises a venous flow pipe (2) from a patient (P) to the reservoir and an arterial flow pipe (3) from the reservoir to the patient, having
- a throttle device (4) for gradually closing, in particular clamping, the venous flow pipe in order to throttle the quantity of venous flow to the reservoir;
- a vacuum device (5) for applying a vacuum to the reservoir in order to increase the quantity of venous flow to the reservoir, and
- a control device (6)
∘ which has a single operating element (7) for fixing of the quantity of venous flow to the reservoir by an operator above or below a basic value fixed by gravity feed, and
∘ which, on account of fixing of the quantity of venous flow to the reservoir by an operator,
- delivers to the throttle device (4) a first control signal for fixing the degree of closure of the venous flow pipe in order to define the extent of throttling of the quantity of venous flow to the reservoir, and
- delivers to the vacuum device (5) a second control signal for fixing the quantity of the vacuum in the reservoir, in order to define the extent of the increase in the quantity of venous flow to the reservoir.

2. Apparatus according to claim 1, **characterised in that** the control device (6) has a display device (8) for visual display of a display value corresponding to the quantity of venous flow.

3. Apparatus according to claim 1 or 2, **characterised in that** the vacuum device (5) is connected by a pipe (11) to a vacuum source or comprises a vacuum source, in particular a pump, in integrated relationship.

4. Apparatus according to any of claims 1, 2 or 3, **characterised in that** a vacuum sensor (12) is provided for detecting the vacuum present in the reservoir and connected to the control device (6) for providing a first measurement signal.

5. Apparatus according to any of the preceding claims, **characterised in that** a level sensor (13) is provided for detecting the level of the quantity of blood present in the reservoir (1) and connected to the control device (6) for providing a second measurement signal.

6. Apparatus according to any of the preceding claims, **characterised in that** an arterial flow quantity sensor (14) is provided for detecting the flow quantity being pumped in the arterial flow pipe (3) and connected to the control device (6) for providing a third measurement signal.

7. Apparatus according to any of the preceding claims, **characterised in that** a blood pump (9) is provided, to which is delivered by the control device (6) a third control signal which fixes the flow rate of the blood pump.

8. Apparatus according to any of the preceding claims, **characterised in that** the single operating element (7) is designed for presetting a quantity of blood to be stored in the reservoir (1).

9. Apparatus according to any of the preceding claims, **characterised in that** the vacuum device (5) comprises a safety device (15) which, upon control by the control device (6) or upon failure of control, interrupts application of the vacuum to the reservoir (1) and produces ambient pressure in the reservoir (1).

## Revendications

1. Dispositif pour la détermination de l'afflux de sang veineux vers un réservoir de sang (1) d'une circulation sanguine extracorporelle comprenant une conduite d'amenée (2) de sang veineux d'un patient (P) au réservoir et une conduite d'amenée (3) de sang artériel du réservoir au patient, avec
- un dispositif de striction (4) pour la fermeture progressive, en particulier par clampage, de la conduite d'amenée de sang veineux, pour restreindre le débit d'amenée de sang veineux vers le réservoir ;
- un dispositif de dépression (5) pour soumettre le réservoir à une dépression, afin d'augmenter le débit d'amenée de sang veineux vers le réservoir, et
- un dispositif de commande (6),
o qui présente un élément de commande (7) unique pour permettre à un opérateur de fixer le débit d'afflux de sang veineux vers le réservoir au-dessus ou en dessous d'une valeur de base définie par le transport par gravité, et
o qui, sur la base de la définition par l'opérateur du débit d'afflux de sang veineux vers le réservoir
- délivre au dispositif de striction (4) un premier signal de commande pour définir l'importance de la fermeture de la conduite d'amenée de sang veineux, afin de déterminer l'étendue de la réduction du débit d'amenée de sang veineux vers le réservoir, et
- qui délivre au dispositif de dépression (5) un deuxième signal de commande pour définir l'importance de la dépression dans le réservoir, afin de déterminer l'étendue de l'augmentation du débit d'amenée de sang veineux vers le réservoir.

2. Dispositif selon la revendication 1, **caractérisé en ce que** le dispositif de commande (6) comporte un dispositif indicateur (8) pour l'affichage visuel d'une valeur indicatrice correspondant au débit d'afflux de sang veineux.

3. Dispositif selon la revendication 1 ou 2, **caractérisé en ce que** le dispositif de dépression (5) est relié par une conduite (11) à une source de vide, ou comprend une source de vide intégrée, en particulier une pompe.

4. Dispositif selon l'une des revendications 1, 2 ou 3, **caractérisé en ce qu'**un capteur de vide (12) est prévu pour déterminer la dépression appliquée dans le réservoir, et **en ce que** celui-ci est relié au dispositif de commande (6) pour l'émission d'un premier signal de mesure.

5. Dispositif selon l'une des revendications précédentes, **caractérisé en ce qu'**un capteur de niveau (13) est prévu pour déterminer le niveau de sang présent dans le réservoir (1), et **en ce que** celui-ci est relié au dispositif de commande (6) pour l'émission d'un deuxième signal de mesure.

6. Dispositif selon l'une des revendications précédentes, **caractérisé en ce qu'**un capteur de débit (14) d'amenée de sang artériel est prévu pour déterminer le débit d'amenée dans la conduite d'amenée de sang artériel (3), et **en ce que** celui-ci est relié au dispositif de commande (6) pour l'émission d'un troisième signal de mesure.

7. Dispositif selon l'une des revendications précédentes, **caractérisé en ce qu'**un pompe à sang (9) est prévue, à laquelle le dispositif de commande (6) délivre un troisième signal de commande fixant le débit de refoulement de la pompe à sang.

8. Dispositif selon l'une des revendications précédentes, **caractérisé en ce que** l'unique élément de commande (7) est prévu pour la fixation d'une quantité de sang à stocker dans le réservoir (1).

9. Dispositif selon l'une des revendications précédentes, **caractérisé en ce que** le dispositif de dépression (5) comprend un dispositif de sécurité (15), lequel interrompt l'application d'une dépression dans le réservoir (1) et applique la pression ambiante dans le réservoir (1) en réaction à une instruction du dispositif de commande (6) ou en cas de défaillance de commande.
